# EUROPEAN PATENT APPLICATION

(11) **EP 4 144 381 A1**
(43) Date of publication of application: **08.03.2023**
(21) Application number: 22180091.5
(22) Date of filing: 21.06.2022
(51) Int. Cl.: A61L 2/10, F21S 8/02

(54) **VEHICLE SURFACE DISINFECTANT SYSTEM AND METHOD**

(30) Priority: 28.06.2021 US 202163215835 P; 11.05.2022 US 202217742134
(71) Applicant: Faiveley Transport Tours, 37701 Saint Pierre des Corps Cedex (FR)
(72) Inventor: Aubin, Philippe, 37701 Saint-Pierre-des-Corps (FR); Brunaux, Yannick, 37701 Saint-Pierre-des-Corps (FR)
(74) Representative: Laufhütte, Dieter

(57) **Abstract**

A vehicle surface disinfectant system (20) includes a lighting module (22) having at least one ultra-violet (UV) light element (26) and at least one visible light element (28). The UV light element(s) (26) and the visible light element(s) (28) may be at least partially separately electrically actuated for the visible light element(s) (28) to emit visible light while the UV light element(s) (26) is off. The lighting module (22) may be inserted into a lighting receptacle in an interior of a vehicle.

## Description

### CROSS-REFERENCE TO RELATED APPLICATIONS

This application claims priority to U.S. Provisional Application No. 63/215,835 (filed 28-June-2021), the entire disclosure of which is incorporated herein by reference.

### BACKGROUND

### Technical Field.

Embodiments of the subject matter described herein relate to transit vehicles. Other embodiments relate to disinfecting interior surfaces of transit vehicles or other types of vehicles.

### Discussion of Art.

Transit vehicles like busses and passenger rail cars are used to carry people from place to place. Other vehicles such as automobiles, vans, sport utility vehicles, and the like, may carry a significant number of passengers as taxis, ride-hailing vehicles, and the like. Considering the typically large number of persons entering, exiting, and traveling inside such vehicles on a daily basis, the vehicle interior surfaces may be subject to microbial and viral contamination. Such contamination can be mitigated by nightly cleaning crews using disinfectant sprays or liquids to clean the interior surfaces. To be even minimally effective, however, such cleaning can take a long time, may require large cleaning crews, and/or is expensive. It is also very difficult to staff such a team during a pandemic period. Effectiveness of such a process that requires repetitive and hard working conditions is also an issue for train operators.

Therefore, it may be desirable to provide a vehicle surface disinfectant or cleaning system and method that differ from existing systems and methods.

### BRIEF DESCRIPTION

In one or more embodiments, a system (e.g., vehicle surface disinfectant system) includes a lighting module having a housing, at least one ultra-violet (UV) light element disposed in the housing, at least one visible light element disposed in the housing, and plural electrical connectors attached to the housing. The at least one UV light element and the at least one visible light element are electrically connected to receive electrical power from the plural electrical connectors and to be separately electrically actuated for the at least one visible light element to emit visible light when the at least one UV light element is off. The housing is configured for insertion into a lighting receptacle in an interior of a vehicle, and the plural electrical connectors are configured to mate with corresponding receptacle connectors in the lighting receptacle when the housing is inserted in the lighting receptacle. In one embodiment, little to no modification of an existing lamp or light installation is required. Instead, the system may simply be swapped or exchanged for the existing lighting system.

In one or more embodiments, a system (e.g., vehicle surface disinfectant system) includes an offboard control unit having a controller and a communication device electrically connected to the controller. The control unit is configured for operable deployment at a location off-board plural vehicle. The control unit is configured to control the communication device to communicate control signals to the vehicles. The control signals are configured to control operation of plural UV light elements in interiors of the vehicles independent of operation of one or more visible light elements in the interiors of the vehicles.

In one or more embodiments, a method (e.g., a method of disinfecting a vehicle interior surface) includes, onboard a vehicle, receiving a control signal from an off-board source. The method further includes, responsive to receiving the control signal, activating at least one UV light element of a lighting module onboard the vehicle. The lighting module includes the UV light element(s) and at least one visible light element both disposed in a common housing that is removable from a lighting receptacle in an interior of the vehicle. The UV light element(s) is activated to emit UV light separately from activation of the at least one visible light element to emit visible light.

In one or more embodiments, a method (e.g., a method of disinfecting a vehicle interior surface) includes, with at least one controller located at a location off-board plural vehicles, communicating control signals to the vehicles. The control signals are configured to control operation of plural UV light elements in interiors of the vehicles independent of operation of one or more visible light elements in the interiors of the vehicles.

### BRIEF DESCRIPTION OF THE DRAWINGS

The inventive subject matter may be understood from reading the following description of non-limiting embodiments, with reference to the attached drawings, wherein below:
FIG. 1 is a schematic view of a first embodiment of a vehicle surface disinfectant system;
FIG. 2 is a schematic view of another embodiment of a vehicle surface disinfectant system;
FIG. 3 is a schematic view of another embodiment of a vehicle surface disinfectant system;
FIG. 4 is a schematic view of another embodiment of a vehicle surface disinfectant system;
FIG. 5 is a schematic view of another embodiment of a vehicle surface disinfectant system; in the illustrated coordinate graphs, the x axes represents time "t" and the y axes represents electronic operational activity (e.g., transceiver transmission output, processing activity, or light output, in watts or the like); and
FIG. 6 is a schematic view of another embodiment of a vehicle surface disinfectant system including UV light sensing and related functionality.

### DETAILED DESCRIPTION

Embodiments of the subject matter described herein relate to systems and methods for disinfecting vehicle interior surfaces using ultra-violet (UV) light. For example, in one embodiment, a system includes a lighting module having a housing, at least one UV light element disposed in the housing, at least one visible light element disposed in the housing, and plural electrical connectors attached to the housing. The housing may be configured for insertion into a lighting receptacle in an interior of a vehicle (e.g., a bus, passenger rail car, airplane, or other transit vehicle, or other vehicle more generally), and the plural electrical connectors are configured to mate with corresponding receptacle connectors in the lighting receptacle when the housing is inserted in the lighting receptacle.

In this manner, according to one aspect of the invention, an existing vehicle light unit (having visible light elements only) can be swapped out for the lighting module, which can then be controllably activated to selectively apply UV light to interior surfaces of the vehicle for disinfection purposes, such as reducing microbial (e.g., bacterial and/or viral) contamination. This can eliminate or reduce the need for additional work and/or expense to be expended when installing the inventive lighting module. For example, in one embodiment, the same sized hole or receptacle in which a pre-existing lamp was disposed may be used to install the inventive lighting module without having to change the size of this hole or receptacle. The conductive bodies (e.g., wires, cables, conductive buses, etc.) supplying power to the pre-existing lamp also may be connected to the UV light element and/or the visible light element to power the same. Optionally, the UV light element may be sized (e.g., sufficiently small) to fit inside the same housing as the pre-existing lamp. For example, the UV light element may be inserted into the same lens or cover in which the pre-existing visible light lamp already is present.

The UV light element and the visible light element are electrically connected to receive electrical power from the plural electrical connectors and to be separately electrically actuated for the at least one visible light element to emit visible light when the at least one UV light element is off. For example, the UV light element can be activated to emit UV light independently of the visible light element. (Visible light refers to white light, or the range of wavelengths of the electromagnetic spectrum that are visible to the human eye. UV light refers to light in the 10-400 nm wavelength range. A specific example of UV light that may be suitable for killing viruses, bacteria, or other microbes is UV-C, in the 100-280 nm wavelength range. UV-A and UV-B, or combinations of different UV light, may also be suitable.)

The UV light element can generate and emit UV light while the visible light element is deactivated and not generating or emitting visible light. Optionally, the UV light element can generate and emit UV light while the visible light element is activated and generating or emitting visible light at the same time. Optionally, a single light element can be provided that can selectively switch between different wavelengths of light or ranges of wavelengths of light that are generated. For example, in a first mode of operation (e.g., illumination without disinfection mode), the light element may generate and emit visible light but not UV light. In a second mode of operation (e.g., a disinfection without illumination mode), the light element may generate and emit UV light but not visible light. In a third mode of operation (e.g., illumination and disinfection mode), the light element may generate both visible light and UV light. Such a multi-purpose light element may have different lamps that can generate different wavelengths of light, and that can be separately controlled to generate the different wavelengths of light.

In another embodiment, the lighting module further includes a control unit, a communication unit, and at least one switch. The communication unit and the switch are operably coupled to the control unit. The control unit is configured to actuate the switch to activate the UV light element(s) for emitting UV light responsive to receiving a designated control signal over the communication unit from an external source, such as an off-board source, another remote source, a source that is onboard the vehicle but outside of the lighting module, or a source that is in the lighting module but outside of the control unit. These sources can represent sensors, another control unit, a dispatch center or facility, or the like.

For example, in another embodiment, the system may include an off-board control unit having a controller and a communication device electrically connected to the controller. The off-board control unit may be at a location off-board plural vehicles (i.e., it is not onboard any vehicle or is not onboard the same vehicle as the lighting module). The off-board control unit also may control the communication device to communicate control signals to the vehicles. The control signals may control operation of plural UV light elements in interiors of the vehicles independent of or separate from operation of one or more visible light elements in the interiors of the vehicles.

Thus, according to one aspect, the UV light element(s) may be remotely actuated, allowing for the provision of safeguards to prevent or limit activation other than under designated conditions.

For example, in one embodiment, the system may be configured for the UV light element(s) to be activated only for discrete designated time periods, with successive activation in each time period being dependent upon receipt of a different designated control signal. The UV light element may be activated for the designated time period responsive to receipt of a first designated control signal, and then is deactivated. Later activation during additional, successive and/or spaced apart designated time periods depends upon receipt of respective additional designated control signals, one for activating the UV light element for each time period. The amount of UV light generated by the UV light element(s) during a single designated time period may be less than a threshold for human safety. However, if the UV light elements are activated for plural of the designated time periods responsive to receiving plural respective designated control signals (from the remote source), the aggregate amount of UV light applied to surfaces within the vehicle, over a time horizon (e.g., one day), is sufficient for killing microbes. Since activation to limited to one designated time period for each control signal, and since the amount of UV light during each time period is under the safety threshold, activation under a failure condition will not be harmful to vehicle occupants, whereas controlled, intended activation is sufficient for at least partial disinfection of vehicle interior surfaces.

In another embodiment, the lighting modules (for deployment onboard plural vehicles) may additionally include respective UV sensors. The UV sensors are configured to detect UV light emitted by the UV light elements, and to generate sensor signals indicative of the detected UV light. Information of the sensor signals (e.g., the sensor signals themselves, or information obtained from the sensor signals) is communicated to the off-board control unit, which may be configured to control a device, e.g., a display screen, to display or otherwise convey the information to users. This may include, for example, displaying which of the UV light elements are emitting UV light above a threshold and which are not, for the vehicles in question, allowing maintenance personal to quickly and easily identify where to replace UV light elements that have failed or are subject to possible failure.

The subject matter described herein provides solutions to the problems or issues associated with disinfecting vehicles that are used over extended periods of time and/or that are used to transport a large number of people. These types of vehicles can be difficult to disinfect due to the little time available for disinfecting the many surfaces inside the vehicles, the number of persons that have been onboard the vehicles, etc. Additionally, these types of vehicles may not be easily taken off-line or out of service for installation of new lighting modules that require cutting of space for the modules, re-wiring or running new wires to power the modules, etc. The subject matter described herein provides solutions to some or all of these problems or issues by describing UV lighting modules that can be quickly and easily retrofitted into existing receptacles, that may use existing wiring for prior, non-UV lighting modules, that may be controlled to generate UV light during the relatively short time periods that no passengers are onboard within the area illuminated by the modules, etc. At least one technical effect of the subject matter described herein destroys or eliminates pathogens, viruses, bacteria, etc. in heavily or repeatedly used vehicles during the short time periods that persons are not onboard and without having to modify the structure or wiring of the vehicles in which the modules are installed.

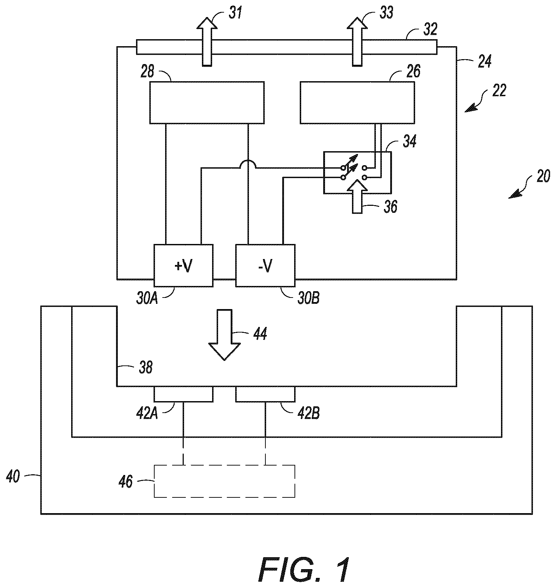

Turning now to FIG. 1, in an embodiment, a vehicle surface disinfection system 20 includes a lighting module 22 having a housing 24, at least one UV light element 26 (also referred to as a disinfecting light element), at least one visible light element 28, and plural electrical connectors 30a, 30b. (Two connectors are shown in the drawings as an example, but there could be more than two.) The light elements and electrical connectors are at least partially disposed in the housing. Generally, the light elements are positioned relative to the housing so that when the light elements are electrically activated, the light elements emit light out of and away from the housing. For this purpose, the housing may include a transparent or translucent side panel 32 (or plural such panels) disposed in a path of the light emitted by the light elements. Alternatively, the housing may include one or more openings disposed in the path of the light emitted by the light elements. Such an opening could be occupied by side, light-emitting surfaces of the light elements themselves, e.g., in instances where the light elements are LEDs (light emitting diodes). Alternatively, the light elements could be disposed on and attached to an exterior surface of the housing (e.g., as in the case of surface-mount LEDs), with electrical lead portions of the light elements extending into the housing.

The UV light element(s) and the visible light element(s) are electrically connected to receive electrical power from the plural electrical connectors. For example, the electrical connectors may be configured for receiving positive voltage (+V) and negative voltage or ground (-V) portions of an electrical power signal, as part of an electrical circuit that includes the light elements. (If there are multiple visible lights elements, they may be connected in parallel to one another to receive the electrical power. Also, if there are multiple UV light elements, they may be connected in parallel to one another to receive the electrical power.) The UV light element and the visible light element are also electrically connected to be at least partially separately electrically activated, for the visible light element to emit visible light 31 when the UV light element is off and thereby not emitting UV light 33. That is, activation of the visible light element does not require concurrent activation of the UV light element.

In the embodiment shown in FIG. 1, the lighting module 22 may include one or more switches 34 electrically interconnected between the UV light element(s) 26 and the electrical connectors 30a, 30b. The switch is configured to be actuated in response to a control input 36 (discussed in more detail below or elsewhere herein), to close an electrical circuit for providing electrical power to the UV light elements. The switch may be a solid-state switch (e.g., transistor circuit), a relay or contactor, or the like. In one embodiment, the switch is configured to remain or transition to an open state (where no power is provided to the UV light element) in the absence of the control input 36 or a loss in electrical power, e.g., by way of a spring or other elastic return element or the like. In the embodiment of this figure, the visible light element is directly connected to the electrical connectors. Here, if electrical power is present at the connectors (e.g., by way of a vehicle's interior lighting being activated generally), the visible light element is activated (turned on) to emit visible light. Concurrently, whether the UV light element is activated (to emit UV light) depends on the state of the switch. Thus, when power is present at the connectors, the visible light element is always on, and the UV light may or may not be on. The two are thereby partially independently or separately controllable, e.g., the UV light element can only be activated when the visible light element is activated, but the visible light element can be active when the UV light element is off. (In this example, the three states of operation are thereby: (i) when no power is present at the connectors, all off; and when power is present at the connectors, either (ii) UV light and visible light or (iii) visible light only.)

According to one aspect, the housing 24 of the lighting module 22 may be configured for insertion into a lighting receptacle 38 in an interior of a vehicle 40. The plural electrical connectors 30a, 30b are configured to electrically contact (e.g., mate with) corresponding receptacle connectors 42a, 42b in the lighting receptacle when the housing is inserted in the lighting receptacle, e.g., as indicated by arrow 44. In other words, the lighting module is configured to plug into the lighting receptacle, for operation of the light elements. (As shown, the receptacle connectors may be electrically connected to a vehicle lighting control and power supply system 46, which controllably supplies electrical power to lights onboard the vehicle.)

The lighting module 22 may be a "drop in" replacement for existing lighting units onboard a vehicle or plural vehicles. That is, the housing 24 may have the same shape as the housings of existing lighting units of a vehicle (or class or group of vehicles), such that it fits into existing lighting receptacles of the vehicle. Also, the pin-out configuration of the electrical connectors 30a, 30b may be the same as that of the existing lighting units, e.g., the same number, placement, and voltage configurations (e.g., positive voltage and negative voltage or ground) of electrical connectors. Also, the lighting module 22 may be configured to operate at the same voltage/power level (e.g., 12V DC) as already provided onboard the vehicle for powering interior lights. Thereby, a vehicle can be provided with UV lights for surface disinfection by swapping out existing lighting units, which only have visible light elements, with the lighting modules as described herein, which incorporate both UV light elements and visible light elements. The lighting modules still provide visible lighting in the interior of the vehicle, and may be configured (in terms of total wattage output of visible light) to provide the same or similar amount of visible light as the lighting units they replace.

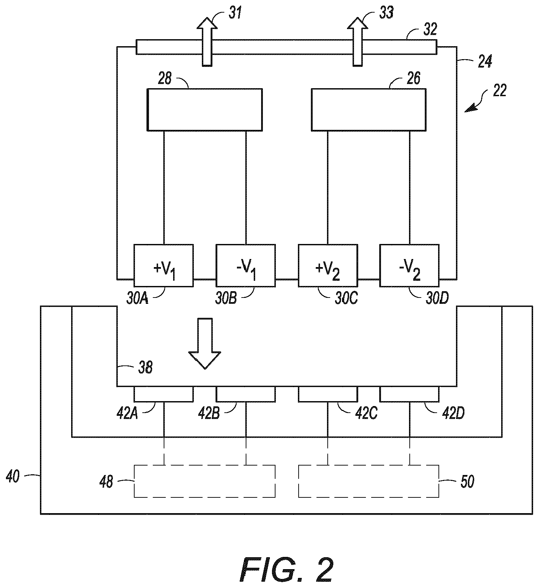

In other embodiments, the visible light element(s) and the UV light element(s) may be completely independently controllable, such that the UV light element may be activated when the visible light element is deactivated, the visible light element may be activated when the UV light element is deactivated, and/or both may be activated or deactivated (turned on or off) concurrently. This could be done, for example, by having a second, separately controlled switch element in association with the visible light element. Alternatively or additionally, with reference to FIG. 2, the light elements 26, 28 could be configured for electrical connection to separate circuits, e.g., via respective electrical connection to different sets of electrical connectors 30a, 30b and 30c, 30d that are in turn respectively connectable to different sets of receptacle connectors 42a, 42b and 42c, 42d, which are in turn respectively electrically connected to different vehicle lighting circuits 48, 50. The light elements would then be separately actuated by way of separate control portions (e.g., respective switches) of the circuits 48, 50.

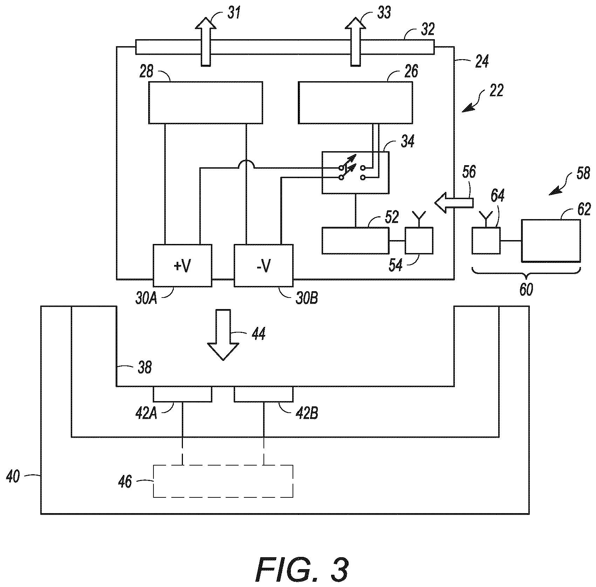

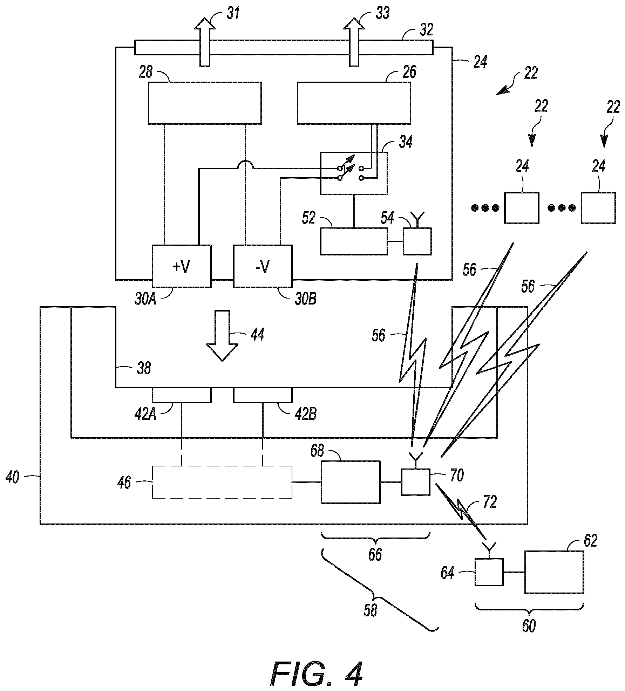

With reference to FIG. 3, in another embodiment, the lighting module 22 may further include a control unit 52 (e.g., circuit having a microprocessor) and a communication unit 54. The communication unit and the switch(es) 34 are operably coupled (e.g., electrically connected) to the control unit. The communication unit may include a wireless transceiver or a wireless receiver that is configured to wirelessly communicate using radio-frequency signals at a designated frequency or within a designated frequency band. The control unit is configured to actuate the switch to activate the UV light element(s) 26 for emitting the UV light 33 responsive to receiving a designated control signal 56 over the communication unit from an off-board source 58 or another remote source (see FIG. 4 and related discussion below).

Although not shown explicitly in FIG. 3, the control unit 52 (and/or the communication unit 54) may be electrically connected to the circuit of the connectors 30a, 30b for receiving electrical power over the connectors when the lighting module is received in the receptacle 38 and power is present at the receptacle connectors 42a, 42b. Also, in embodiments where the lighting module includes plural switches, one of which is electrically connected between the visible light element(s) and the connectors 30a, 30b, the control unit 52 could also be configured to control activation and deactivation of the visible light element, based on additional designated control signals.

As noted, the control unit 52 activates the UV light element responsive to receiving the designated control signal 56. The control unit may be configured to only activate the UV light element upon receiving the correct signal, that is, receipt of the designated signal is a pre-requisite for activating the UV light element, and if the designated signal is not received, the UV light element cannot be activated. If the system is configured for the control signal to originate with the offboard or other remote source, this may serve as a failsafe, whereby the UV light element cannot be activated locally, or cannot be activated solely locally. That is, in one embodiment the UV light element is activated only responsive to the control unit receiving the designated control signal from the offboard or other remote source. In another embodiment, the UV light element is activated only responsive to both the control unit receiving the designated control signal from the offboard or other remote source and the control unit receiving another, second designated control signal, e.g., a local actuation signal initiated by maintenance personnel at the vehicle. In either case, as discussed in more detail below, the offboard or other remote source 58 may be set up to generate the designated control signal 56 based on safety information and/or other information it receives about the vehicle, e.g., vehicle location, vehicle speed, vehicle schedule, the presence or absence of persons in the interior of the vehicle, time of day, sound or image data of the interior of the vehicle or the exterior of the vehicle, and so on. For example, the offboard or remote source may generate the control signal responsive to the vehicle being in a location where passengers typically are not onboard the vehicle (e.g., in a storage facility, parking facility, maintenance facility, etc.). The offboard or remote source may generate the control signal responsive to the vehicle having no speed or being stationary for a designated period of time (and/or where the control signal may be generated after a designated delay to provide passengers an opportunity to disembark). The offboard or remote source may generate the control signal responsive to the vehicle schedule indicating that the vehicle is not scheduled to be transporting passengers. The offboard or remote source may generate the control signal responsive to the presence or absence of persons in the interior of the vehicle (e.g., based on output from a sensor that detects the presence of people inside the vehicle, such as a camera, motion sensor, etc.). The offboard or remote source may generate the control signal responsive to the time of day being within a designated range of times associated with passengers not being onboard the vehicle (e.g., between 1 am and 4am). The offboard or remote source may generate the control signal responsive to sounds detected in the vehicle being quieter than a designated decibel level (e.g., indicating that it is likely that no passengers are onboard). The offboard or remote source may generate the control signal responsive to an image or video of the interior of the vehicle indicating or showing that no one is onboard the vehicle where the lighting module is located. The offboard or remote source may generate the control signal responsive to an image or video of the exterior of the vehicle indicating that no one is onboard the vehicle (e.g., by looking through windows of the vehicle). Additionally or alternatively, one or more of these control signals may be generated by one or more components onboard the vehicle, such as one or more of the sensors 98 described herein.

In an embodiment, the remote source 58 includes an offboard control unit 60 having a controller 62 (e.g., circuitry with one or more microprocessors) and a communication device 64 (e.g., radio or other wireless communication device) electrically connected to the controller. The control unit 60 is configured for operable deployment at a location off-board plural vehicles, e.g., when deployed the control unit is not onboard any vehicle(s). For example, the control unit may be located at a dispatch center, a maintenance depot, or the like. Operable deployment at such a location may include being configured to run on electrical power available at the location, and with access to appropriate communications infrastructure (e.g., Internet and/or public telecommunications) for the communication device 64 to communicate with onboard communication units, e.g., unit 54. The offboard control unit 60 is configured to control the communication device 64 to communicate control signals 56 to the vehicle 40. As noted above, the control signals may control operation of the UV light element(s) in the interior of the vehicle at least partially independent of operation of the visible light element(s) in the interior of the vehicle.

In a transportation network, plural vehicles (e.g., busses, airplanes, automobiles, etc.) or vehicle systems (e.g., passenger trains, convoys of separate vehicles, an articulated bus formed from interconnected sections, etc.) may be outfitted with the onboard portions of the system (e.g., the lighting modules) as set forth herein. In such a case, the offboard control unit 60 may be configured to communicate respective control signals to all the vehicles that are so equipped, to activate the UV light elements on board the vehicles under designated and controlled conditions. Optionally, the control unit may be onboard the vehicle or vehicle system and may control activation of the UV light elements.

Turning to FIG. 4, in another embodiment, the remote source 58 may include a vehicle control unit 66 having a vehicle control unit controller 68 (e.g., circuit with a microprocessor) and a vehicle control unit communication device 70 electrically connected to the controller. The vehicle control unit is positioned onboard the vehicle 40, but is not housed in a lighting module. Instead, the vehicle control unit may be located, for example, in a central control cabinet that houses all or part of the vehicle lighting control and power supply system 46, and/or other vehicle electronics (such as HVAC control). The vehicle control unit is configured to wirelessly communicate with the offboard control unit 60, and to communicate with plural lighting modules 22 onboard the vehicle 40. In an embodiment, communication with the lighting modules is wireless. In other embodiments, alternatively or additionally, communication may be via a wired connection, e.g., plural cables extending between the location of the vehicle control unit and the lighting receptacles. The vehicle control unit may receive wireless signals from one or more sources. The vehicle control unit is configured to assess the received signals relative to designated criteria. The vehicle control unit is configured to generate and communicate the control signals 56 (for activating the UV light elements) responsive to if any of the received signals meet designated criteria. For example, the offboard control unit 60 may communicate first control signals 72 to the vehicle control unit 66. The signals 72 may be encoded signals generated and communicated by the offboard control unit authorizing activation of the UV light elements onboard the vehicle. The vehicle control unit determines that the signals 72 are valid for authorizing/controlling activation of the UV light elements, e.g., by way of comparing the coding/information of the signals 72 to information about valid codes (i.e., the designated criteria) stored in a data unit (memory) of the vehicle control unit.

The embodiment of FIG. 4 may allow for centralizing or partially centralizing certain processor- or transceiver-intensive operations, as opposed to outfitting each lighting module with suitable equipment for such operations. For example, the vehicle control unit communication device 70 may have a relatively large transceiver power output for communicating with offboard sources, thereby obviating the need for each lighting module to have such a transceiver. Instead, the lighting modules may have lower-power transceivers, for onboard communications only. As another example, the vehicle control unit communication device may have a transceiver with particular circuitry for communicating over long distances, e.g., a cellular communications transceiver for communicating over public wireless networks, whereas the lighting modules may have a different type of transceiver for onboard or close-range communications only, e.g., Wi-Fi. In either or both cases, the lighting modules can thereby be physically smaller and/or more cost effective to implement and power.

As another example, the vehicle control unit controller 68 may have a larger processing capacity than the lighting module control units 52 individually. Again, this may allow the lighting modules to be physically smaller and/or more cost effective to implement and power. For example, the signals 72 communicated from the offboard control unit 60 may have a greater degree of encoding or complexity, or there may be more signals 72 communicated more often, than the signals 56 communicated between the vehicle control unit 66 and the lighting module control units 52. By having a larger processing capacity, the vehicle control unit controller is able to timely process such signals, whereas the lighting module control units may not need as much processing capacity to timely process the intra-vehicle signals 56.

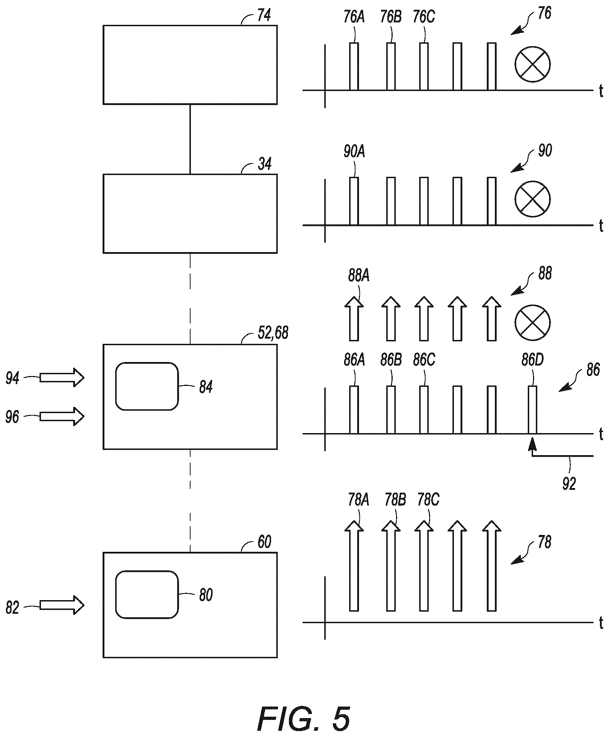

FIG. 5 illustrates another embodiment of the system, which is configured for the UV light elements 74 (box 74 in FIG. 5 represents the UV light elements 26 generally) to be activated only for discrete designated time periods 76 (individually shown as 76a, 76b, 76c, etc.), with successive activation in each time period being dependent upon receipt of respective, different designated control signals 78 (individually shown as 78a, 78b, 78c, etc.) In other words, for a given vehicle, UV light elements are activated for the designated time period 76a responsive to receipt of a first designated control signal 78a, and then are deactivated. Later activation for additional, successive, spaced apart designated time periods 76b, 76c, etc. depends upon receipt of respective additional designated control signals 78b, 78c, etc., one for activating the UV light elements for each time period 76. If there is a failure mode or condition (e.g., receipt of an errant signal), this prevents the UV light elements from being activated for more than the relatively short time period of the designated time period 76, as a safety precaution.

To explain in more detail, according to one aspect, the offboard control unit 60 is programmed with a process or algorithm 80 (e.g., stored in a memory unit) by which the offboard control unit generates and transmits the control signals 78 under designated circumstances or criteria. Specifically, the offboard control unit receives or is otherwise provided with information 82 about a vehicle outfitted with the UV light elements 74. The information may include sensor information of or from the vehicle (as explained elsewhere herein), scheduling information of the vehicle, time-of-day and calendar date, control inputs from a user operating a user interface of the offboard control unit, etc. Responsive to the information as compared to designated criteria of the algorithm 80, the offboard control unit may generate the control signals 78. For example, the offboard control unit may be programmed or otherwise configured to generate the control signals responsive to the information indicating that the vehicle is parked in a maintenance shed/depot or other location where the vehicle is not supposed to be carrying passengers. Such information could include a sensed location of the vehicle (e.g., via GPS or cameras), a time-of-day that corresponds to when the vehicle is scheduled to be at the maintenance shed/depot, a sensed movement condition indicating the vehicle is stationary, etc. As another example, the information could be sensor information indicating the vehicle interior is not currently occupied, e.g., from a sensor such as a camera, motion sensor, sound sensor, door sensor, and so on.

When the offboard control unit generates a first of the control signals 78a, it communicates it to an onboard controller, e.g., the lighting module control unit 52 or the vehicle control unit controller 68. The onboard controller receives the signal. The onboard controller is programmed with a process or algorithm 84 (stored in a memory unit) by which the onboard controller assesses the received signal at 86a, relative to criteria stored in the memory, to determine if the received signal is a designated control signal authorizing or controlling activation of the UV light elements 74. For example, the signal may include a code, and the onboard controller may have information of the code stored in memory. If the code of the received signal matches the information in memory, the onboard controller determines the signal is a designated control signal. Subsequently, the onboard controller generates an electrical signal, at 88a, to actuate the switch or switches 34. The switches are turned on for a time period 90a that corresponds to the designated time period 76a, which results in the UV light elements being activated and emitting UV light for the designated time period 76a. The onboard controller is configured to generate the electrical signal 88a for the designated time period and then to deactivate the electrical signal to turn off the switch and deactivate the UV light elements. The onboard controller is configured so that subsequent activation of the UV light elements requires receipt of subsequent designated control signals, e.g., which may be different than the first designated control signal. For example, the offboard control unit 60 may generate and communicate subsequent control signals 78 (e.g., 78b 78c, and so on), each of which is received by the onboard controller and sequentially assessed at 86 (e.g., 86b, 86c, and so on, which represent processing cycles/time), e.g., the onboard controller may be configured to compare codes contained in the signals to respective information of the codes stored in a memory accessible to the onboard controller. Each time there is a match, the onboard controller applies subsequent electrical signals 88 to the switches 34, which are turned on for subsequent, sequential time periods 90, which results in activation of the UV light elements for the subsequent, sequential designated time periods 76. Between each time period 76, the UV light elements are deactivated (off) and thereby do not emit UV light.

The onboard controller (e.g., 52 or 68) is configured so that if it receives a signal 92 (from whatever source) that does not match the designated criteria (e.g., stored in memory) for assessing whether the signal is a designated control signal for activating the UV light elements, the onboard controller will not generate signals to actuate the switches to activate the UV light elements. That is, the onboard controller is configured, responsive to determining a received signal is not a designated control signal (based on a comparison to designated criteria stored in memory or otherwise), as at 86d, to leave the switches unactuated and the UV light elements thereby off, as indicated by the encircled "X's" in FIG. 5.

In an embodiment, the signal generation algorithm 80 of the offboard control unit 60 may include a cyclic code generator, e.g., a mathematical formula that can generate many secure codes in relatively quick succession. The signal assessment algorithm 84 of the onboard controller (e.g., 52 or 68) includes a corresponding cyclic code generator, which is synchronized with that of the offboard control unit (e.g., through handshake or other initialization signals). Thereby, each time the offboard control unit generates and communicates a new code, the onboard controller generates the same code, and this is used as the designated criteria for assessing receipt of authorized commands to activate the UV light elements.

In another embodiment, the offboard control unit 60 may be configured to generate a set number of multiple control signals 78 each with different designated signal content, with the multiple control signals being cyclically re-used over time. For example, the offboard controller could have a list (e.g., stored in memory) of sixteen or more codes, with the onboard controller (e.g., 52 or 68) having the same list stored in memory. For successively activating (pulsing) the UV light elements, the offboard control unit would sequentially transmit the multiple control signals in the stored list, spaced apart over time. Once at the end of the list, for additionally pulsing the UV light elements, the offboard control unit would re-transit the control signals, spaced apart over time, starting again at the beginning of the list. The onboard controller would similarly assess received signals relative to the order of the list, and relative to designated criteria for each signal of the list.

In another embodiment, the offboard control unit 60 may be configured to generate multiple control signals 78 each with the same code, other encoded content, or other designated signal content, i.e., the same designated signal content transmitted in multiple spaced-apart iterations over time. Here, the onboard controller (e.g., 52 or 68), upon receiving a signal, would assess the signal relative to designated criteria that are indicative of the designated signal content. If the received signal matched the designated criteria, it would indicate that the received signal was a designated control signal for activating the UV light elements for a single designated time period 76. If not, the onboard controller would not activate the UV light elements. Because the onboard controller is configured to only activate the UV light elements for a single time period for each received designated signal, in a fault or error condition, e.g., a mis-assessed signal, or receipt of an errant signal, the UV light elements would only be unintentionally activated for one time period, below the designated threshold for safety.

In addition to assessing the signals 78, 92 (received wirelessly or otherwise) that may be designated control signals (for activating the UV light elements), the onboard controller (e.g., 52 or 68) may be further configured to generate the electrical signals 88 to actuate the switches only responsive to one or more other signals 94, 96 (received over other communication channels, e.g., other wireless connections, other wireless channels, wired connections, etc.) meeting one or more additional criteria. That is, the onboard controller would only actuate (turn on) the switches responsive to determining that the signals received from an offboard source (e.g., signals 78) are designated control signals and that the one or more other signals 94, 96 meet the additional criteria. For example, the first signal 94 may be generated by a secure, manual "UV light" actuation switch onboard the vehicle (e.g., in a locked central control/electronics cabinet), which can only be accessed by maintenance or crew personnel onboard the vehicle. Thus, when the vehicle is at a maintenance depot, the maintenance personnel would ensure the vehicle is empty of passengers, and once it was so determined, the manual switch would be turned on. Subsequently, if designated control signals were received from the offboard source while the manual actuation switch was turned on, the onboard controller would activate the UV light elements, e.g., pulsed activation, as set forth herein. As another example, a second signal 96 could originate from one or more sensors onboard or offboard the vehicle, with the additional criteria relating to, for example, vehicle occupancy status, vehicle location, vehicle schedule information, vehicle movement, and so on.

According to one aspect, the time periods 76 may all be the same length. Additionally, the time periods may be relatively short, such that each activation of the UV light elements is in-effect a pulse or strobe. For example, the length of the time period(s) may be selected so that the amount of UV light generated by the UV light elements 74 within the interior region of the vehicle during a single designated time period 76 is less (or much less) than a designated threshold for human safety. For example, if a designated maximum safe level ofUV-C light exposure for humans is 6mJ/cm2/day (which is atypical value), the designated time period 76 may be selected so that UV light generated during the time period in the vehicle is from 0.5mJ/cm2 to 2mJ/cm2, e.g., 1mJ/cm2. Meaning, if the UV light elements in the vehicle happened to be activated for one cycle (one designated time period) due to a failure or fault condition, while a person was errantly in the vehicle, the person would be exposed only to a safe level of UV light. The exact length of the time period 76 could be selected, on a vehicle type-by-vehicle type basis, as a function of one or more of the following: (i) rated UV light power output of the UV light elements (e.g., in watts); (ii) actual UV light power output of the UV light elements; (iii) rated or actual flux output of the UV light elements (e.g., watts per cm2 at 1 meter); (iv) positioning (e.g., distance) of the UV light elements relative to where passengers would be closest to the light elements under designated, normal circumstances (e.g., standing on the vehicle interior floor, or sitting on passenger seating in the vehicle interior); and/or (v) the selected designated threshold (e.g., 1mJ/cm2).

According to one aspect, if the UV light elements are activated for plural of the designated time periods 76 responsive to receiving plural respective designated control signals 78, the aggregate amount of UV light applied to surfaces within the vehicle, over a designated time horizon (e.g., one day), is sufficient for killing microbes (e.g., viruses or bacteria). Since activation to limited to one designated time period for each control signal, and since the amount of UV light during each time period is under the safety threshold, activation under a failure condition will not be harmful to vehicle occupants, whereas controlled, intended activation for multiple time periods over a designated time horizon is sufficient for at least partial disinfection of vehicle interior surfaces.

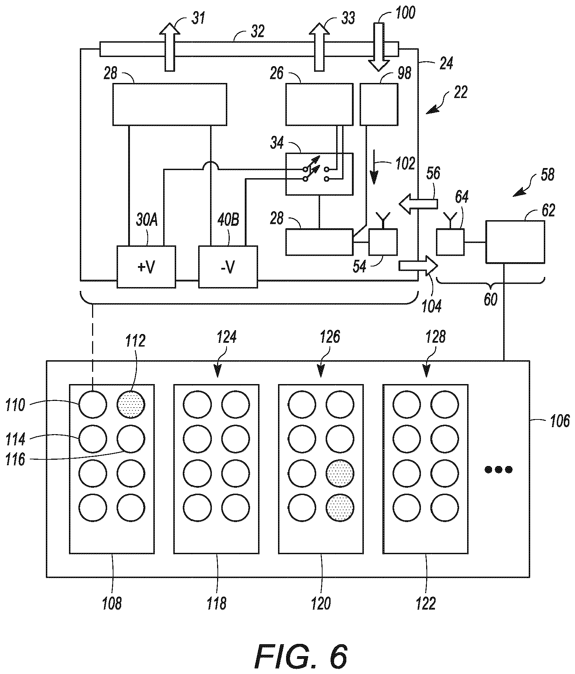

With reference to FIG. 6, in another embodiment, the lighting modules 22 may additionally include respective UV sensors 98. That is, each lighting module may include one or more UV sensors 98. The UV sensor is electrically connected to the control unit 52. The UV sensor is configured to detect incident UV light 100, that is, UV light that falls on the sensor. Since the lighting module is deployed inside a vehicle, the incident UV light will mostly originate from the UV light 33 that is emitted by the UV light element(s) 26. The UV sensor may be positioned to directly receive light from the UV light element, and/or to receive UV light that is reflected off vehicle interior surfaces. The UV sensor is configured to generate sensor signals 102 indicative of the detected UV light, e.g., indicative of a magnitude of the sensed UV light over time. The sensor signals 102 are routed to the control unit 52. The control unit is configured to communicate information 104 of the sensor signals (e.g., the sensor signals themselves, or information obtained from the sensor signals, either continuously or periodically) to the off-board control unit 60. The off-board control unit 60 may be configured to control a device 106, e.g., a display screen, to display or otherwise convey the information 104 to users. For example, the off-board control unit 60 may be configured to control a display screen 106 to display a graphical representation 108 of a vehicle in which the lighting module 22 is housed and deployed. The control unit 60 may also be configured to control the display screen to display a graphical representation 110 of the lighting module, and to change how the lighting module graphical representation is displayed as a function of the received information 104. For example, if the received information 104 indicates the UV light element(s) of the lighting module are emitting UV light above a designated threshold (e.g., and thereby functioning as intended), the control unit may display the lighting module graphical representation in one manner, e.g., a green color, open circle, first designated icon, or other designated representation, to represent correct functioning. On the other hand, if the received information 104 indicates the UV light element(s) of the lighting module are not emitting UV light above the designated threshold (e.g., and thereby may be "burned out" or otherwise not functioning as intended), the control unit may display the lighting module graphical representation in a different, second manner, e.g., a red color, opaque circle, second designated icon, or other second designated representation, to represent incorrect functioning. Thereby, maintenance personnel or other persons can view the graphical representations to quickly understand and identify that the lighting element may need replacement or other servicing.

Optionally, one or more of these sensors may be sense the presence or absence of passengers onboard the vehicle. For example, at least one of the sensors 98 can be a motion sensor, a structured light array sensor, an infrared or thermal sensor, a camera, or the like. Output from this type of sensor may indicate whether there is a person onboard the vehicle (and the UV light elements cannot be activated) or whether there is no person onboard the vehicle (and the UV light elements can be activated). The sensor can provide this output to the control unit (which may be onboard or off-board the vehicle), and the control unit can accordingly control the UV light elements. Optionally, the sensor can provide the output to the UV light elements, which activate or deactivate based on the output. This can prevent the UV light elements from generating UV light that can be harmful to people while passengers are onboard the vehicle.

In another aspect, the off-board control unit 60 may be in communication with plural different lighting modules 22 onboard one vehicle, and it may be configured to receive respective UV-sensor originated information 104 from each of the plural different lighting modules onboard the one vehicle. In such an instance, the off-board control unit may be further configured to additionally control a device to convey respective information about all the plural lighting modules. For example, the off-board control unit may be configured to control a display screen 106 to display plural additional graphical representations 112, 114, 116, etc. of each lighting module, in association with the graphical representation 108 of the vehicle in which the lighting modules are housed and deployed. The off-board control unit may control how each graphical representation 112, 114, 116, etc. is displayed (e.g., as either a first designated representation or a second designated representation, or otherwise) as a function of the received information, e.g., to indicate whether the lighting module is functioning as intended, or to indicate some other quality or aspect of the lighting module. The relative positioning of each graphical representation (relative to the displayed vehicle representation) may be indicative of the location or position of each lighting module onboard the vehicle, and/or the off-board control unit may be further configured to additionally display additional information uniquely identifying each lighting module and/or its position onboard the vehicle. Again, this allows maintenance personnel or other persons to quickly understand and identify which, if any, of plural lighting modules onboard a particular vehicle may need replacing or other servicing.

In another aspect, the off-board control unit 60 may be in communication with plural different lighting modules 22 onboard plural vehicles, and it may be configured to receive respective UV-sensor originated information 104 from all the plural different lighting modules onboard all the plural vehicles. In such an instance, the off-board control unit may be further configured to additionally control a device to convey respective information about all the plural lighting modules of all the plural vehicles. For example, the off-board control unit may be configured to control a display screen 106 to display plural additional graphical representations 118, 120, 122, etc. of each of the vehicles it is in communication with, and/or a selectable subset thereof, e.g., one vehicle at a time, or plural vehicles at the same time but fewer than the total number of vehicles the control unit is in communication with. The off-board control unit may be further configured to control the display screen to concurrently display plural additional graphical representations 124, 126, 128, etc. of the plural lighting modules onboard each vehicle, in association with the displayed graphical vehicle representations. As described above, each of the plural additional graphical representations of the plural lighting modules can be displayed to convey designated information about the subject lighting module. For example, for a given lighting module, its associated displayed graphical representation could be displayed in one manner to reflect one state of the lighting module (or other aspect or characteristic of the lighting module), e.g., functioning to emit UV light above a designated threshold, and in a different, other manner to reflect another state, e.g., not functioning to emit light above a designated threshold. For each displayed vehicle representation, the relative positioning of the graphical representations of the lighting modules associated with the vehicle may be indicative of the locations or positions of the lighting modules onboard the vehicle, and/or the off-board control unit may be further configured to additionally display additional information uniquely identifying each lighting module and/or its position onboard the vehicle. The multiple lighting module representations in conjunction with the multiple vehicle representations allows maintenance personnel or other persons to quickly understand and identify which, if any, of plural lighting modules onboard plural vehicles may need replacing or other servicing, thereby facilitating efficient fleet servicing.

The display screen or other device 106 may be located, for example, at a maintenance depot or maintenance facility, at a dispatch center, at a central office, etc., or it may be incorporated into a portable electronic device such as a laptop, tablet, or smart phone. Device control may be direct or indirect, in whole or in part through the Internet or another network. For example, the off-board control unit could be configured to control a display screen attached to it locally by a cable. Or, the off-board control unit could be configured to communicate information to a website, which in turn would communicate the information to user devices of authorized users linked to the website over the Internet or another network. The off-board control unit may be configured to convey information in multiple different ways, e.g., via a local device directly connected to the off-board control unit and through Internet communications with authorized users.

In one example, a system (e.g., vehicle surface disinfectant system) is provided. The system may include a lighting module having at least one UV light element and at least one visible light element, characterized in that the UV light element(s) and the visible light element(s) may be at least partially separately electrically actuated for the visible light element(s) to emit visible light while the UV light element(s) is off. The lighting module may be inserted into a lighting receptacle in an interior of a vehicle.

The lighting module may be a replacement of an existing lighting module in a receptacle of the vehicle that includes an electrical connection for the existing lighting module. The lighting module may be placed into the same receptacle and connected to the same electrical connection without modifying the receptacle or the electrical connection, with the existing lighting module having no UV light element. The lighting module also may include a control unit, a communication unit, and at least one switch. The communication unit and the at least one switch may be operably coupled to the control unit. The control unit may actuate the at least one switch to activate the at least one UV light element for emitting UV light responsive to receiving a designated control signal over the communication unit from an external source. The communications unit may include a wireless transceiver that receives the designated control signal over a wireless communications channel from the external source. The external source may include a control server at a dispatch center. The control unit my actuate the at least one switch for successive spaced-apart designated time periods responsive to successively receiving different codes as the designated control signal from the external source. During the designated time periods, the at least one UV light element may be activated for emitting UV light. Between the designated time periods, the at least one UV light element may be off. The UV light emitted by each of the at least one UV light element in each of the designated time periods may be individually being less than a designated threshold for safe exposure of humans to the UV light, relative to a closest distance between the at least one UV light element and designated positions of humans as passengers in the interior of the vehicle. Application of the UV light to interior surfaces of the vehicle during an aggregate of the designated time periods over a designated time horizon may be or is sufficient to reduce microbial contamination of the interior surfaces.

The control unit may receive a sensor signal from a sensor onboard the vehicle. The control unit may control activation of the at least one UV light element responsive to the sensor signal meeting one or more designated criteria. The sensor signal may indicate one or more of sound within the interior of the vehicle, movement within the interior of the vehicle, movement of the vehicle, a location of the vehicle, an acceleration of the vehicle, actuation of a door of the vehicle, a presence of one or more persons onboard the vehicle, and/or an absence of the one or more persons onboard the vehicle.

The sensor signal may include a video signal of the interior of the vehicle. The control unit may at least one of communicate information of the video signal to the external source for the external source to generate the control signal based at least in part on the information of the video signal and/or change a number of the successive spaced-apart designated time periods the at least one UV light is activated. The control unit may at least one of control opening of a door of the vehicle and/or activate at least one warning device in the interior of the vehicle concurrently with or just prior to activation of the at least one UV light element. For example, the door may be opened and/or warning device activated at the same time that the UV light element is activated or within a time period that is no longer than required for the transmission of electronic signals between detecting activation of the light element and the opening of the door or activation of the warning device. The control unit may actuate the at least one switch to activate the at least one UV light element for emitting UV light only responsive to both receiving the designated control signal over the communication unit from the external source and receiving a second control signal from a user-actuated input.

The lighting module also may include at least one UV sensor that generates a UV sensor signal indicative of UV light emitted by the at least one UV light element. The lighting module also may include a control unit and a communication unit operably coupled to the control unit. The control unit may control the communication unit to communicate information of the UV sensor signal to an external source. The lighting module may include a housing in which the at least one UV light element and the at least one visible light element are disposed.

In another example, another system (e.g., another disinfectant system) may include an offboard control unit that includes a controller and a communication device electrically connected to the controller. The control unit may be deployed at a location that is off-board plural vehicles. The offboard control unit may control the communication device to communicate first control signals to the vehicles. The first control signals may control operation of plural UV light elements in interiors of the vehicles at least partially separate of operation of one or more visible light elements in the interiors of the vehicles.

The offboard control unit may receive sensor signals over the communication device from the plural vehicles. The sensor signals include information of UV light emitted by the UV light elements. The offboard control unit may generate second control signals to control at least one user interface device to convey the information of the UV light to a user. At least one user interface device may include a display screen. The second control signals may control the display screen to display graphical representations of the vehicles and graphical representations of at least one of the information of the UV light or the UV light elements. The first control signals may include, for each of the vehicles, a respective cyclic code for activating the UV light elements onboard the respective vehicle for successive spaced-apart designated time periods. During the designated time periods the UV light elements are activated for emitting UV light and between the designated time periods the UV lights are off, and cumulative UV light emitted by the UV light elements in the vehicle during each of the designated time periods may be less than a designated threshold for safe exposure of humans to the UV light.

In another example, a method (e.g., for disinfecting a vehicle interior) may include receiving a control signal from an external source at a control unit of a lighting module onboard a vehicle, and, responsive to receiving the control signal at the control unit, activating at least one UV light element of the lighting module. The lighting module may have the at least one UV light element and at least one visible light element both disposed in a common housing that is removable from a lighting receptacle in an interior of the vehicle. The at least one UV light element may be activated to emit UV light separately from activation of the at least one visible light element to emit visible light.

The singular forms "a", "an", and "the" include plural references unless the context clearly dictates otherwise. "Optional" or "optionally" means that the subsequently described event or circumstance may or may not occur, and that the description may include instances where the event occurs and instances where it does not. Approximating language, as used herein throughout the specification and claims, may be applied to modify any quantitative representation that could permissibly vary without resulting in a change in the basic function to which it may be related. Accordingly, a value modified by a term or terms, such as "about," "substantially," and "approximately," may be not to be limited to the precise value specified. In at least some instances, the approximating language may correspond to the precision of an instrument for measuring the value. Here and throughout the specification and claims, range limitations may be combined and/or interchanged, such ranges may be identified and include all the sub-ranges contained therein unless context or language indicates otherwise.

This written description uses examples to disclose the embodiments, including the best mode, and to enable a person of ordinary skill in the art to practice the embodiments, including making and using any devices or systems and performing any incorporated methods. The claims define the patentable scope of the disclosure, and include other examples that occur to those of ordinary skill in the art. Such other examples are intended to be within the scope of the claims if they have structural elements that do not differ from the literal language of the claims, or if they include equivalent structural elements with insubstantial differences from the literal language of the claims.

## Claims

1. A system comprising:
a lighting module having at least one ultra-violet (UV) light element and at least one visible light element,
wherein the at least one UV light element and the at least one visible light element are configured to be at least partially separately electrically actuated for the at least one visible light element to emit visible light while the at least one UV light element is off,
wherein the lighting module is configured for insertion into a lighting receptacle in an interior of a vehicle.

2. The system of claim 1, wherein the lighting module is a configured to be a replacement of an existing lighting module in a receptacle of the vehicle that includes an electrical connection for the existing lighting module, the lighting module configured to be placed into the same receptacle and connected to the same electrical connection without modifying the receptacle or the electrical connection, the existing lighting module having no UV light element.

3. The system of claim 1, wherein the lighting module further comprises a control unit, a communication unit, and at least one switch, wherein the communication unit and the at least one switch are operably coupled to the control unit, the control unit configured to actuate the at least one switch to activate the at least one UV light element for emitting UV light responsive to receiving a designated control signal over the communication unit from an external source.

4. The system of claim 3, wherein the communications unit comprises a wireless transceiver configured to receive the designated control signal over a wireless communications channel from the external source, the external source including a control server at a dispatch center.

5. The system of claim 3, wherein the control unit is configured to actuate the at least one switch for successive spaced-apart designated time periods responsive to successively receiving different codes as the designated control signal from the external source.

6. The system of claim 5, wherein, during the designated time periods, the at least one UV light element is activated for emitting UV light and, between the designated time periods, the at least one UV light element is off, the UV light emitted by each of the at least one UV light element in each of the designated time periods individually being less than a designated threshold for safe exposure of humans to the UV light, relative to a closest distance between the at least one UV light element and designated positions of humans as passengers in the interior of the vehicle.

7. The system of claim 6, wherein application of the UV light to interior surfaces of the vehicle during an aggregate of the designated time periods over a designated time horizon is sufficient to reduce microbial contamination of the interior surfaces.

8. The system of claim 5, wherein the control unit is configured to receive a sensor signal from a sensor onboard the vehicle, the control unit conifgured to control activation of the at least one UV light element responsive to the sensor signal meeting one or more designated criteria.

9. The system of claim 8, wherein the sensor signal is indicative of one or more of sound within the interior of the vehicle, movement within the interior of the vehicle, movement of the vehicle, a location of the vehicle, an acceleration of the vehicle, actuation of a door of the vehicle, a presence of one or more persons onboard the vehicle, or an absence of the one or more persons onboard the vehicle.

10. The system of claim 8, wherein the sensor signal comprises a video signal of the interior of the vehicle, and the control unit is configured to at least one of:
communicate information of the video signal to the external source for the external source to generate the control signal based at least in part on the information of the video signal; or
change a number of the successive spaced-apart designated time periods the at least one UV light is activated.

11. The system of claim 3, wherein the control unit is configured to at least one of control opening of a door of the vehicle or activate at least one warning device in the interior of the vehicle concurrently with or just prior to activation of the at least one UV light element, or wherein the control unit is configured to actuate the at least one switch to activate the at least one UV light element for emitting UV light only responsive to both receiving the designated control signal over the communication unit from the external source and receiving a second control signal from a user-actuated input.

12. The system of claim 1, wherein the lighting module further comprises at least one UV sensor configured to generate a UV sensor signal indicative of UV light emitted by the at least one UV light element, or wherein the lighting module further comprises a control unit and a communication unit operably coupled to the control unit, and wherein the control unit is configured to control the communication unit to communicate information of the UV sensor signal to an external source.

13. The system of claim 1, wherein the lighting module includes a housing in which the at least one UV light element and the at least one visible light element are disposed.

14. A system comprising:
an offboard control unit comprising a controller and a communication device electrically connected to the controller, the control unit configured for operable deployment at a location that is off-board plural vehicles;
the offboard control unit configured to control the communication device to communicate first control signals to the vehicles, the first control signals configured to control operation of plural ultra-violet (UV) light elements in interiors of the vehicles at least partially separate of operation of one or more visible light elements in the interiors of the vehicles, or wherein the offboard control unit is configured to receive sensor signals over the communication device from the plural vehicles, the sensor signals including information of UV light emitted by the UV light elements; and
the offboard control unit configured to generate second control signals to control at least one user interface device to convey the information of the UV light to a user, wherein preferrebly the at least one user interface device includes a display screen, and the second control signals are configured to control the display screen to display graphical representations of the vehicles and graphical representations of at least one of the information of the UV light or the UV light elements, or wherein the first control signals include, for each of the vehicles, a respective cyclic code for activating the UV light elements onboard the respective vehicle for successive spaced-apart designated time periods, wherein during the designated time periods the UV light elements are activated for emitting UV light and wherein between the designated time periods the UV lights are off, and wherein cumulative UV light emitted by the UV light elements in the vehicle during each of the designated time periods is less than a designated threshold for safe exposure of humans to the UV light.

15. A method comprising:
onboard a vehicle, receiving a control signal from an external source at a control unit of a lighting module onboard the vehicle; and
responsive to receiving the control signal at the control unit, activating at least one ultra-violet (UV) light element of the lighting module, the lighting module having the at least one UV light element and at least one visible light element both disposed in a common housing that is removable from a lighting receptacle in an interior of the vehicle, wherein the at least one UV light element is activated to emit UV light separately from activation of the at least one visible light element to emit visible light.
